# EUROPEAN PATENT SPECIFICATION

(11) **EP 2 526 117 B1**
(45) Date of publication and mention of the grant of the patent: **06.05.2015**
(21) Application number: 11701494.4
(22) Date of filing: 20.01.2011
(51) Int. Cl.: C07K 14/50, C12P 21/00

(54) **PROCESS FOR PREPARING FGF-21 WITH LOW DEGREE OF O-GLYCOSYLATION**
VERFAHREN ZUR HERSTELLUNG VON FGF-21 MIT GERINGER O-GLYKOSYLIERUNG
PROCÉDÉ DE PRÉPARATION DE FGF-21 PRÉSENTANT UN FAIBLE DEGRÉ DE O-GLYCOSYLATION

(30) Priority: 18.06.2010 US 356086 P; 15.06.2010 EP 10165927; 22.01.2010 US 692227; 22.01.2010 WO PCT/EP2010/050720
(43) Date of publication of application: 28.11.2012
(73) Proprietor: Novo Nordisk A/S, 2880 Bagsværd (DK)
(72) Inventor: SLAABY, Rita, DK-2880 Bagsværd (DK); LAUTRUP-LARSEN, Inger, DK-2880 Bagsværd (DK)
(86) International application number: PCT/EP2011/050718
(87) International publication number: WO 2011/089170

(56) References cited:
- WO-A1-2009/105357
- WO-A2-2006/028595
- WO-A2-2010/084169
- US-A1- 2002 068 325
- TAKAMATSU SHINJI ET AL: "Monitoring of the tissue distribution of fibroblast growth factor containing a high mannose-type sugar chain produced in mutant yeast", GLYCOCONJUGATE JOURNAL, vol. 20, no. 6, 2003, pages 385-397, XP002597174, ISSN: 0282-0080

## Description

### FIELD OF THIS INVENTION

This invention relates to a special process for preparing Fibroblast Growth Factor 21 (FGF21) and analogues thereof and aspects closely related thereto.

### BACKGROUND OF THIS INVENTION

Fibroblast growth factors are polypeptides expressed in developing and adult tissues. They are involved in several physiological mechanisms including for example metabolic regulation and cellular differentiation. A whole family of more than twenty fibroblast growth factors exists (the FGF family). Three members of the FGF family including FGF19, FGF21, and FGF23 form a subfamily functioning as endocrine factors involved in metabolic regulation.

Fibroblast Growth Factor 21 or FGF-21, herein for short FGF21, is expressed preferentially in the liver and has been shown to exert hormone-like metabolic effects.

In decades, it has been possible to prepare a large range of peptides and proteins recombinantly, for example in a bacterium such as *Eschericia Coli* or in yeast such as *Saccharomyces cerevisiae.* Proteins expressed in yeast are often O-glycosylated. In pharmaceutical proteins, O-glycosylation should usually be avoided. For example, human FGF21 is highly O-glycosylated when prepared recombinantly in yeast.

FGF21 muteins which are mutated at several positions in its sequence and having reduced capacity for O-glycosylation when expressed in yeast are disclosed in WO 2006/028595 A2, WO 2009/105357 A1 relates to modified host cells for producing recombinant glycoproteins that have reduced O-glycosylation by deletion of several genes, including PMT genes.Claim 1 in US 2002/0068325 relates to a method of producing a heterologous protein in fungi comprising: providing a recipient fungi cell wherein the quality control mechanism in said cell is modified so that incompletely folded heterologous proteins are not degraded in the endoplasmic reticulum; and introducing to said recipient fungi cell a polynucleotide expression construct. According to claim 8 therein, the recipient cell is modified in the mannosyltransferase gene comprising a gene selected from the group consisting of *PMT1, PMT2, PMT3, PMT4, PMT5* and *PMT6*. FGF is not dealt with in said US patent application.

### OBJECTS OF THIS INVENTION

The object of this invention is to overcome or ameliorate at least one of the disadvantages of the prior art, or to provide a useful alternative.

Another aspect of this invention relates to the furnishing of a process for effectively preparing FGF21 and analogues thereof.

Another aspect of this invention relates to the furnishing of a recombinant process for preparing FGF21 and analogues thereof having a low degree of O-glycosylation or no O-glycosylation.

Another aspect of this invention relates to the furnishing of a recombinant process in which the degradation of FGF21 and analogues thereof is decreased.

### DEFINITIONS

The sequence of the native human FGF21 protein is available from the UNIPROT database with accession no. Q9NSA1 and is as follows: MDSDETGFEHSGLWVSVLAG-LLLGACQAHPIPDSSPLLQFGGQVRQRYLYTDDAQQTEAHLEIREDGTVGGAADQSPESLLQL-KALKPGVIQILGVKTSRFLCQRPDGALYGSLHFDPEACSFRELLLEDGYNVYQSEAHGLPLHLPGNK-QILGVKTSRFLCQRPDGALYGSLHFDPEACSFRELLLEDGYNVYQSEAHGLPLHLPGNK-SPHRDPAPRGPARFLPLPGLPPALPEPPGILAPQPPDVGSSDPLSMVGPSQGRSPSYAS. The 209 amino acid precursor protein includes a signal peptide (amino acids 1-28) and a mature protein (amino acids 29-209). The mature human protein is included herein as SEQ ID NO:1 (amino acids 1-181), and the signal peptide as SEQ ID NO:2 (amino acids 1-28).

The term "analogue" as referred to herein in the context of FGF21, i.e., an FGF21 analogue, refers to a polypeptide that is or can be, deduced or derived from native human FGF21, from SEQ ID NO:1 in particular, by modification of the amino acid sequence thereof. Such modification, amendment or change may include substitution, deletion, and/or addition of one or more amino acids. In total, preferably not more than 8, more preferred not more than 6 amino acids are substituted, deleted and/or added. For example, amino acids may be added and/or deleted at the C-terminus, at the N-terminus, or internally in the amino acid sequence. Preferably amino acids are added and/or deleted at the C- and/or N-terminus, more preferably at the N-terminus. Amino acid sequences with C- or N-terminally deleted amino acids may also be referred to as truncated sequences, as is known in the art. Likewise, amino acids added internally in the sequence may be referred to as insertions. The term FGF21 analogue refers to compounds having glucose lowering effect. The glucose lowering effect can be determined as described in WO 2010/0841169, especially example 8 relating to a potency assay for glucose uptake in 3T3-L1 adipocytes. Said FGF21 analogues have a identity with human FGF21 which is at least 95%. The identity can be determined as described in WO 2010/084169, especially pages 10 & 11.

Herein, the term "PMT" (Protein Mannosyl Transferase) covers enzymes which attach the first mannose on serine or threonine residues in proteins leading to an O-glycosylation mannose tree. However, it is not all serine and threonine residues which are O-glycosylated in a protein. There are at least 6 enzymes in the PMT family (Pmtp1-6). They are grouped in three sub families each group represented by Pmt1p, Pmt2p or Pmt4p.

Herein, the term "knock out" covers deletion of the open reading frame. The deletion can be either a deletion of the open reading frame or substitution of the open reading frame with a marker. With a 'marker' is meant genes used in classical yeast genetics for deletion of genes and substitution with another gene which activity can be selected for such as *TRP1* or *KanMX4.*

In one embodiment, all the amino acids and amino acid residues dealt with herein are amino acids and amino acid residues which can be coded for by the triplon ("codon") of nucleotides and, hence, the corresponding FGF21 analogues can be prepared recombinantly.

Herein, the term "PMT1" covers Protein-Mannosyl-transferase 1.

Herein, the term "PMT2" covers Protein-Mannosyl-transferase 2.

Herein, the term "PMT4" covers Protein-Mannosyl-transferase 4.

Herein, the term "PEP4" covers protein peptidase A.

Herein, the term "YPS1" covers the YPS1-encoeded aspartic protease yapsin 1 (also called YAP3).

Herein, the term "MKC7" covers glycosyl-phosphatidylinositol-linked aspartyl protease 7. Herein, the term "TRP1" covers Phosphoribosylanthranilate Isomerase 1.

Herein, the term "*kanMX*" is used for the gene from Tn903 that confers resistance to the aminoglycosid antibiotic G418 of transformed yeasts.

Herein, the term "marker" is used for genes which gene product activity can be selected for.

### BRIEF DESCRIPTION OF THIS INVENTION

Briefly, the present invention is as described in the claims and clauses below.

FGF21 and analogues thereof, e.g., analogues with N-terminal amino acid extensions, can be expressed in *Saccharomyces cerevisiae*. These N-terminal amino acid extensions can consist of up to about 20 amino acids, preferably up to about 15 amino acids and all the amino acids are amino acids which can be coded for by the triplon and prepared recombinantly. In the present invention, this requires strain design in which a strain disrupted in *PMT2, PEP4* and *YPS1* is created. This strain can be designed using classical techniques relying on homologous recombination allowing specific integration at the respective loci. FGF21 and analogues thereof, e.g., analogues with N-terminal extension or mutations, are coded for on an S. *cerevisiae* expression vector which can be maintained in *S. cerevisiea*. To direct the FGF21 analogue to the secretory pathway a pre-pro sequence including a signal peptide (for example the MFalpha pre-pro leader sequence) may be provided in the recombinant expression vector. This sequence is joined to the DNA encoding the FGF21 analogue in correct reading frame. This signal peptide ensures secretion to the media. Upstream and adjacent to the FGF21 analogue sequence a dibasic amino acid sequence is placed ensuring cleavage of the prepro sequence from the FGF21 analogue before secretion to the media. The cleavage is likely to be caused by Kex2p activity. The FGF21 analogue can be harvested from the media.

Surprisingly, the inventors of the present invention have found that the problem with O-glycosylated FGF21 and analogues thereof when wild type yeast was used was solved by disruption of *PMT2* in the expression strain. By deletion of *PMT2* from the wild type yeast strain O-glycosylation of FGF21 and analogues thereof was decreased.

### DETAILED DESCRIPTION OF THIS INVENTION

Expression of FGF21 in a wild type *S. cerevisiae* yeast strain has proven to be problematic as the FGF21 and analogues thereof are highly O-glycosylated. This invention describes the solution to this problem by expressing FGF21 and analogues thereof in a *pmt2* knock out yeast strain. This is a strain where the host *PMT2* gene encoding Protein-Mannosyl-transferase 2 (Pmt2p) has been eliminated in a way that is non-reversible.

In wild type yeast, the gene encoding Pmt2p can be disrupted using classical techniques relying on homologous recombination allowing specific replacement of the wildtype *PMT2* gene by integration of a deletion allele into the *PMT2* locus, using another gene as selectable marker. The selectable marker is a gene, which activity can be selected for such as *TRP1, KanMX4* or other markers.

FGF21 and analogues thereof can subsequently be expressed from expression plasmids in this knock out yeast strain resulting in reduced O-glycosylation. It is advantageously to combine the *pmt2* knock-out with protease knock-outs in *PEP4, YPS1 (YAP3)* and *MKC7 (YPS2)* genes in order to avoid degradation of the expressed protein.

Generally, the DNA is inserted into an expression vector, such as a plasmid, in proper orientation and correct reading frame for expression. The vector is then introduced into the host through standard techniques and, generally, it will be necessary to select for transformed host cells.

Host cells that have been transformed by the recombinant DNA used in this invention are then cultured for a sufficient period of time and under appropriate conditions known to those skilled in the art in view of the teachings disclosed herein to permit the expression and secretion of FGF21 and analogues thereof to be produced according to the method of the invention. The skilled art worker is able to select a culture medium and proper culturing conditions and to recover FGF21 from the medium after the cultivation.

Useful yeast plasmid vectors include the cPOT (Kjeldsen et al. in Gene 170: 107-112, 1996) and YEp13, YEp24 (Rose and Broach in Methods in Enzymol. 185: 234-279, 1990), and pG plasmids (Schena et al. in Methods in Enzymol. 194: 289-398,1991).

Methods for the transformation of S. *cerevisiae* include the spheroplast transformation, lithium acetate transformation, and electroporation, cf. Methods in Enzymol. 194 (1991). Preferably the transformation is as described in the examples herein.

Suitable promoters for *S. cerevisiae* include the *MFα1* promoter, galactose inducible promoters such as *GAL1, GAL7* and *GAL10* promoters, glycolytic enzyme promoters including *TPI1* and *PGK1* promoters, *TRP1* promoter, CYC1 promoter, *CUP1* promoter, *PHO5* promoter, *ADH1* promoter, and HSP promoter. A suitable promoter in the genus *Pichia* is the *AOX1* (methanol utilisation) promoter.

The transcription terminal signal is preferably the 3' flanking sequence of a eukaryotic gene which contains proper signal for transcription termination and polyadenylation. Suitable 3' flanking sequences may, e.g., be those of the gene naturally linked to the expression control sequence used, i.e., corresponding to the promoter.

The DNA constructs that are used for providing secretory expression of the desired FGF21 or analogue thereof comprise a DNA sequence that includes a leader sequence linked to the polypeptide by a yeast processing signal. The leader sequence contains a signal peptide ("pre-sequence") for protein translocation across the endoplasmic reticulum and optionally contains an additional sequence ("pro-sequence"), which may or may not be cleaved within yeast cells before the polypeptide is released into the surrounding medium. Useful leaders are the signal peptide of S. *cerevisiae* MFα1p, MFα1*, Yap3p, Bar1p, Hsp150p and *S. kluyveri* MFα signal peptides and prepro-sequences of *S. cerevisiae* MFα1, Yap3p, Prep, Hsp150p, and *S. kluyveri MFα* and synthetic leader sequences described in WO 92/11378, WO 90/10075 and WO 95/34666.

The DNA sequence encoding the desired FGF21 or analogue thereof may be of genomic or cDNA origin, for instance be obtained by preparing a genomic or cDNA library and screening for DNA sequences coding for all or part of the polypeptide by hybridization using synthetic oligonucleotide probes in accordance with standard techniques (see, for example, Sambrook, J, Fritsch, EF and Maniatis, T in Molecular Cloning: A Laboratory Manual, Cold Spring Harbor Laboratory Press, New York, 1989). The DNA sequence encoding FGF21 or an analogue thereof may also be prepared synthetically by established standard methods, *e.g.* the phosphoamidite method described by Beaucage and Caruthers in Tetrahedron Letters 22 (1981), 1859 - 1869, or the method described by Matthes et al. in EMBO Journal 3 (1984), 801 - 805. The DNA sequence may also be prepared by polymerase chain reaction using specific primers, for instance as described in US 4,683,202 or by Saiki et al. in Science 239 (1988), 487 - 491.

Expression of FGF21 or analogues thereof in this *pmt2* knock out strain did not change the expression level in any substantial amount compared with the expression level using a non-*pmt2* knock out strain but, as mentioned herein, reduced the O-glycosylation significantly.

In one embodiment of this invention, the expression of FGF21 or analogues thereof is performed using a yeast strain that has knock-outs of *YPS1, PEP4* and *PMT2.*

In one embodiment of this invention, the expression of FGF21 or analogues thereof is performed using a yeast strain that has knock-outs of *YPS1, PEP4* and *MKC7.*

In one embodiment of this invention, the expression of FGF21 or analogues thereof is performed using a yeast strain that has knock-outs of *YPS1, PEP4, MKC7* and of *PMT2.*

In one embodiment of this invention, the FGF21 analogue contains Ala or His in position 17 and/or 36. Hereby, the degree of degradation is lowered.

If the task is to prepare human FGF21, the process of this invention is to be performed at conditions at which the ratio between O-glycosylated FGF21 and non-O-glycosylated FGF21 obtained is below about 20%, preferably below about 10%, more preferred below about 5% and even more preferred below about 2% (weight/weight). Similarly, this applies if the task is to prepare an analogue of FGF21.

### PREFERRED FEATURES OF THIS INVENTION

To sum up and supplement the above statements, the features of this invention are as defined in the claims.

The following examples are offered by way of illustration, not by limitation.

### Example

### General procedures.

All expressions plasmids are of the cPOT type, similar to those described in EP 171,142. These are 2µ-based expression vectors characterized by containing the *Schizosaccharomyces pombe* triose phosphate isomerase gene (POT) for the purpose of plasmid selection and stabilization in S. *cerevisiae.* The plasmids also contain the S. *cerevisiae* triose phosphate isomerase promoter and terminator. These sequences are similar to the corresponding sequences in plasmid pKFN1003 (described in WO 90/10075). In order to facilitate cloning of different fusion proteins the DNA sequence encoding the *MFα1* pre-pro leader has been changed to incorporate a *Ncol* site and is called the *MFα1** pre-pro leader. Thus the *NcoI-XbaI* fragment is simply replaced by an *NcoI-XbaI* fragment encoding the FGF21 construct or that of an analogue thereof. Such *NcoI-XbaI* fragments may be synthesized using synthetic oligonucleotides and PCR according to standard techniques. In addition to the alpha-leader other leaders can be used.

Yeast transformants and derivatives thereof were prepared by transformation of the host S. *cerevisiae* strains. The yeast strains were grown in liquid YPGGE (1% Bacto yeast extract, 2% Bacto peptone, 2% galactose, 2% glycerol, 1 % ethanol) to an O.D. at 600 nm of 0.6. 100 ml of culture were harvested by centrifugation, washed with 10 ml of water, recentrifuged and resuspended in 10 ml of a solution containing 1.0 M sorbitol, 25 mM Na₂EDTA pH 8.0 and 6.7 mg/ml dithiotreitol. The suspension was incubated at 30°C for 15 minutes, centrifuged and the cells resuspended in 10 ml of a solution containing 1.0 M sorbitol, 10 mM Na₂EDTA, 0.1 M sodium citrate, pH 5.8, and 2 mg Glucanex 200G (Novozyms). The suspension was incubated at 30°C for 30 minutes, the cells collected by centrifugation, washed in 10 ml of 1.2 M sorbitol and 10 ml of CAS (1.0 M sorbitol, 10 mM CaCl₂, 10 mM Tris HCl pH 7.5) and resuspended in 2 ml of CAS. For transformation, 1 ml of CAS-suspended cells was mixed with approximately 0.1 mg of plasmid DNA and left at room temperature for 15 minutes. 1 ml of (20% polyethylene glycol 4000, 10 mM CaCl₂, 10 mM Tris HCl, pH 7.5) was added and the mixture left for a further 30 minutes at room temperature. The mixture was centrifuged and the pellet resuspended in 0.1 ml of SOS (1.2 M sorbitol, 33% v/v YPD, 6.7 mM CaCl₂) and incubated at 30°C for 2 hours. The suspension was then centrifuged and the pellet resuspended in 0.5 ml of 1.2 M sorbitol. Then, 6 ml of top agar (the SC medium of Sherman et al. (1982) in Methods in Yeast Genetics, Cold Spring Harbor Laboratory) containing 1.2 M sorbitol plus 2.5% agar at 52°C was added and the suspension poured on top of plates containing the same agar-solidified, sorbitol containing medium.

### Example 1

### pmt1::KanMX4, pmt2::KanMX4 and pmt4::KanMX4 gene disruptions in ME1487

A yeast strain BY4741-pmt2 with the *PMT2* gene deleted and the genotype *Mat a his3D1 leu2D0 lys2D0 ura3D0 yal023c::kanMX4*, was purchased from the Euroscarf deletion strain collection. In this yeast strain, the DNA fragment encoding the *PMT2* open reading frame has been replaced with the *KanMX4* dominant selectable marker, conferring resistance to the antibiotic G418/geneticin (Wach et al. Yeast 10 (1994)1793-1808.

Genomic DNA was isolated from BY4741 and used as template in a standard PCR reaction with synthetic oligonucleotides oILLa-0056: CCGTTTCGTGTACTGTTTA and oILLa-0079: GGCTAAAGGGTTCAAGAAAT in order to amplify a synthetic DNA fragment containing the *Δpmt2::KanMX4* deletion allele cassette. This DNA fragment was used directly for transformation of ME1487: *MATα, tpi::LEU2 pep4-3 leu2 ura3 yps1::URA3* (Egel-Mitani et al. Enzyme and Microbial Technology 26 (2000), 671-677) containing the cPOT plasmid expressing FGF21. Transformation of yeast was done by the lithium acetate / single-stranded carrier DNA / polyethylene glycol method *(*Methods in Enzymology: 350, 87 - 96). After transformation, the yeast cells were plated on YEPD (1% yeast extract, 2% peptone, 2% dextrose) agar and incubated overnight at 30°C followed by replicaplating onto selective YEPD agar + 200 mg/L G418. Yeast colonies appearing after 3 days further incubation at 30°C were isolated and characterized by PCR which verified the correct integration of *pmt2::KanMX4* allele into the *PMT2* locus and thus confirmed the replacement of the wild type allele with the deletion allele. The genotype of the resulting strain, yDNP-255, was *MATα, tpi1::LEU2 pep4-3 leu2 ura3 yps1::URA3 pmt2::kanMX4.* yDPN-255 contained the cPOT plasmid expressing FGF21.

Similarly, the locus *PMT1* and *PMT4* were disrupted creating the strains:
yDNP-257: *MATα, tpi1::LEU2 pep4-3 leu2 ura3 yps1::URA3 pmt1::kanMX4* and
yDNP-260: *MATα, tpi1::LEU2 pep4-3 leu2 ura3 yps1::URA3 pmt4::kanMX4*. Both containing the cPOT plasmid expressing FGF21.

### Example 2

### pmt2:: TRP1-FA gene disruption in NNY574

The auxotrophic marker, the *trp1-FA* deletion, was introduced into a *S.cerevisiae* bagground strain to facilitate classical one-step knock out of relevant genes, as described by Horecka and Jigami (Horecka and Jigami Yeast 15 (1999) 1769-1774; The *trp1-FA* designer deletion for PCR-based gene functional analysis in Saccharomyces cerevisiae. Yeast 15, 1769-1774). The fragment deleted from the *TRP1* locus corresponded exactly to a cassette termed *TRP1-FA* carried in many yeast vectors and was composed of 141 bp from the 5'-untranslated region followed by the *TRP1* open reading frame followed by 50 bp from the 3'-untranslated region. In order to introduce the *trp1-FA* deletion, the strain of interest was transformed with a PCR fragment containing the fused 5'- and 3'-untranslated regions from further upstream and downstream the *TRP1-FA* region for a classical one-step deletion of *TRP1-FA.* Deletion of the *TRP1-FA* region was verified by PCR. This strain had then *PMT2* gene deleted by transformation with a DNA fragment consisting of the TRP1-FA cassette flanked by the *pmt2* 5'-untranslated region and the *pmt2* 3'-untranslated region, followed by selection for the TRP1-FA marker phenotype. Recombination of the *pmt2::TRP1-FA* allele into the *PMT2* locus resulted in replacement of the wildtype *PMT2* gene with *pmt2::TRP1-FA* allele. Isolated yeast cells were characterized by classical yeast genetic methods. Correct integration was confirmed by PCR.

### Example 3

Western blot analysis was used to determine the effect of *pmt1, pmt2* and *PMpmt4* knock out on the expression of FGF21 compared to a wild type strain. Samples of media from the cultivated yeast strains secreting FGF21 were run on 4-12% BisTris SDS page in Mes-SDS buffer. The proteins in the gel were transferred to nitrocellulose and immunostained with polyclonal FGF21 antibody goat FGF21 (Santa Cruz sc-16842), and secondary donkey anti goat IgG-HRP (Santa Cruz sc-2020). For detection the chemifluorescence detection method was used. The result was that in the wild trype strain FGF21 migrated as a double band. When *PMT4* was deleted it had no effect. When *PMT1* was deleted it has some effect but when *PMT2* was deleted FGF21 migrated as a single band and O-glycosylation was significantly decreased.

### Example 4

Deletion of the *PMT2* gene from the host can be used for secretory expression of FGF21 analogues in S. *cerevisiae.* In the FGF21 analogues, amino acids can be amended or changed, substituted, deleted, and/or one or more amino acids can be added or combinations thereof. When expressing the FGF21 analogues in the *S.cerevisiae* host with deletion of the *PMT2* gene, O-glycosylation is decreased significantly. This can be observed by Western blot analysis of the growth medium from cultivation of the *S. cerevisiae* strain secreting FGF21 or FGF21 analogues, as described in Example 3. FGF21 and analogues thereof migrate as a double band when secreted from a wild type strain. This is changed when expression is from a *pmt2 knock out S. cerevisae* host. Then, human FGF21and the FGF21 analogue will migrate as a single band.

For FGF21 analogue expression the desired sequence was inserted in the cPot expression vector and transformed into the *pmt2* knock out strain described in Example 3 above. FGF21 analogues comprising the following mutations, insertions and/or extensions were made:
1) -1G, K56R, K59R, K69R, K122R
2) -5G, -4S, -3G, -2S, -1G, K56R, K59R, K69R, D102E, N121Q, K122R, M168L
3) -14E, -13E, -12A, -11E, -10A, -9G, -8G, -7A, -6G, -5G, -4S, -3G, -2G, -1S, K56R, K59R, K69R, K122R
4) -15E, -14E, -13S, -12A, -11A, -10S, -9G, -8A, -7A, -6A, -5G, -4S, -3A, -2A, -1A, K56R, K59R, K69R, K122R)
5) K56R, K59R, K69R, K122R)
6) -5G, -4S, -3G, -2S, -1G, K56R, K59R, K69R, K122R)
7) -1G

The supernatants from yeast cultures secreting the above compounds were analysis on Western blot analysis as described in Example 3. It was observed that only a single band visualized with polyclonal FGF21 antibody appeared on the Western blot and not a double band as FGF21 in a wild type S. *cerevisiae* strain confirming the effect of deletion of *pmt2* in the expression strain.

The nomenclature used for the above compounds is as described in WO 2010/084169, especially at pages 6-11.

### Example 5

LC-MS analysis using a LC/MSD TOF instrument (Agilent) is used to determine the molecular mass of secreted peptides in yeast supernatants according to the settings recommended by the manufacturer. Deconvolution of the TIC chromatogram is done using the accompanying software. The abundances of full-length and O-glycosylated species is obtained from the deconvoluted spectrum and used for estimation of the percentage of full-length peptide vs the pool of full-length and O-glycosylated FGF21.

The citation and incorporation of patent documents herein is done for convenience only and does not reflect any view of the validity, patentability, and/or enforceability of such patent documents. The mentioning herein of references is no admission that they constitute prior art.

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Herein, the word "comprise" is to be interpreted broadly meaning "include", "contain" or "comprehend" (*vide*, EPO guidelines C, III, 4.13).

## Claims

1. A process for recombinant expression of native FGF21 with SEQ ID NO:1 and analogues thereof which analogues can be deduced or derived from native human FGF21 with SEQ ID NO:1 by modification of the amino acid sequence said modification being substitution, deletion and/or addition of one or more amino acids which analogues have glucose lowering effect and which analogues have an identity with human FGF21 which is at least 95%, in yeast wherein the yeast used is a *pmt2* knock out strain.

2. A process according to claim 1, wherein an FGF21 analogue with an N-terminal extension is prepared, said extension consisting of up to about 20 amino acids.

3. The process according to claim 1, wherein the yeast used is *Saccharomyces cerevisae*.

4. The process according to claim 1 or 2, wherein the yeast used is a *pep4* knock out strain.

5. The process according to any one of the preceding claims, wherein the yeast used is an *yps1* knock out strain.

6. The process according to any one of the preceding claims, wherein the yeast used is an *mkc7* knock out strain.

7. The process according to any one of the preceding claims, wherein the yeast is cultivated at conditions at which the exogenous DNA sequence is expressed and the desired FGF21 or an analogue thereof as defined in claim 1 is recovered.

8. The process according to any one of the preceding claims, wherein said FGF21 is secreted into the culture medium.

9. The process according to any one of the preceding claims, wherein the knock out *pmt2* yeast strain is prepared using the *TRP1* marker or *KanMX4* marker, preferably the *TRP1* marker.

10. The process according to any one of the preceding claims, wherein the ratio between O-glycosylated FGF21 or a derivative thereof, on one hand, and non-O-glycosylated FGF21 or an analogue thereof as defined in claim 1, on the other hand, is below about 20%, preferably below about 10%, more preferred below about 5% and even more preferred below about 2% (weight/weight).

11. A process according to any one of wherein the FGF21 analogue prepared has been mutated at position R17 and/or R36.

## Patentansprüche

1. Verfahren zur rekombinanten Expression von nativem FGF21 mit der SEQ ID NO:1 und Analogen davon, wobei sich die Analogen von nativem menschlichem FGF21 mit der SEQ ID NO:1 durch Modifikation der Aminosäuresequenz ab- oder herleiten lassen, wobei es sich bei der Modifikation um Substitution, Deletion und/oder Addition einer oder mehrerer Aminosäuren handelt, wobei die Analogen eine glucosesenkende Wirkung haben und eine Identität mit menschlichem FGF21 aufweisen, die wenigstens 95% beträgt, in Hefe, wobei es sich bei der verwendeten Hefe um einen *pmt2*-knock-out-Stamm handelt.

2. Verfahren nach Anspruch 1, wobei ein FGF21-Analog mit einer N-terminalen Verlängerung hergestellt wird, wobei die Verlängerung aus bis zu 20 Aminosäuren besteht.

3. Verfahren nach Anspruch 1, wobei es sich bei der verwendeten Hefe um *Saccharomyces cerevisae* handelt.

4. Verfahren nach Anspruch 1 oder 2, wobei es sich bei der verwendeten Hefe um einen *pep4*-knock-out-Stamm handelt.

5. Verfahren nach einem der vorhergehenden Ansprüche, wobei es sich bei der verwendeten Hefe um einen *yps1*-knock-out-Stamm handelt.

6. Verfahren nach einem der vorhergehenden Ansprüche, wobei es sich bei der verwendeten Hefe um einen *mkc7*-knock-out-Stamm handelt.

7. Verfahren nach einem der vorhergehenden Ansprüche, wobei die Hefe bei Bedingungen kultiviert wird, bei denen die exogene DNA-Sequenz exprimiert wird, und das gewünschte FGF21 oder ein Analog davon mit der in Anspruch 1 angegebenen Bedeutung gewonnen wird.

8. Verfahren nach einem der vorhergehenden Ansprüche, wobei das FGF21 in das Kulturmedium sezerniert wird.

9. Verfahren nach einem der vorhergehenden Ansprüche, wobei der Knock-out-*pmt2*-Hefestamm unter Verwendung des *TRP1*-Markers oder *KanMX4*-Markers, vorzugsweise des *TRP1*-Markers, hergestellt wird.

10. Verfahren nach einem der vorhergehenden Ansprüche, wobei das Verhältnis zwischen O-glykosyliertem FGF21 oder einem Derivat davon einerseits und nicht O-glykosyliertem FGF21 oder einem Analog davon mit der in Anspruch 1 angegebenen Bedeutung andererseits unterhalb etwa 20 Gew.-%, vorzugsweise unterhalb etwa 10 Gew.-%, stärker bevorzugt unterhalb etwa 5 Gew.-% und noch stärker bevorzugt unterhalb etwa 2 Gew.-% liegt.

11. Verfahren nach einem der vorhergehenden Ansprüche, wobei das hergestellte FGF21-Analog an Position R17 und/oder R36 mutiert wurde.

## Revendications

1. Procédé pour l'expression recombinante de FGF21 natif avec SEQ ID NO: 1 et des analogues de celui-ci, lesdits analogues pouvant être déduits ou dérivés de FGF21 humain natif avec SEQ ID NO: 1 par modification de la séquence d'acides aminés, ladite modification étant une substitution, une délétion et/ou une addition d'un ou plusieurs acides aminés, lesdits analogues ayant un effet hypoglycémiant et lesdits analogues ayant une identité avec FGF21 humain qui est d'au moins 95 %, dans la levure, la levure utilisée étant une souche knock out pour *pmt2.*

2. Procédé selon la revendication 1, dans lequel un analogue de FGF21 avec une extension N-terminale est préparé, ladite extension étant constituée de jusqu'à environ 20 acides aminés.

3. Procédé selon la revendication 1, dans lequel la levure utilisée est *Saccharomyces cerevisae.*

4. Procédé selon la revendication 1 ou 2, dans lequel la levure utilisée est une souche knock out pour *pep4.*

5. Procédé selon l'une quelconque des revendications précédentes, dans lequel la levure utilisée est une souche knock out pour *yps1.*

6. Procédé selon l'une quelconque des revendications précédentes, dans lequel la levure utilisée est une souche knock out pour *mkc7.*

7. Procédé selon l'une quelconque des revendications précédentes, dans lequel la levure est cultivée dans des conditions dans lesquelles la séquence d'ADN exogène est exprimée et le FGF21 souhaité ou un analogue de celui-ci tel que défini dans la revendication 1 est récupéré.

8. Procédé selon l'une quelconque des revendications précédentes, dans lequel ledit FGF21 est sécrété dans le milieu de culture.

9. Procédé selon l'une quelconque des revendications précédentes, dans lequel la souche de levure knock out pour *pmt2* est préparée en utilisant le marqueur *TRP1* ou le marqueur *KanMX4,* de préférence le marqueur *TRP1.*

10. Procédé selon l'une quelconque des revendications précédentes, dans lequel le rapport entre FGF21 O-glycosylé ou un dérivé de celui-ci, d'une part, et FGF21 non O-glycosylé ou un analogue de celui-ci tel que défini dans la revendication 1, d'autre part, est inférieur à environ 20 %, de préférence inférieur à environ 10 %, plus préférablement inférieur à environ 5 % et encore plus préférablement inférieur à environ 2 % (poids/poids).

11. Procédé selon l'une quelconque des revendications précédentes dans lequel l'analogue de FGF21 préparé a été muté à la position R17 et/ou R36.
